# EUROPÄISCHE PATENTANMELDUNG

(11) **EP 0 827 722 A2**
(43) Veröffentlichungstag der Anmeldung: **11.03.1998**
(21) Anmeldenummer: 97114027.2
(22) Anmeldetag: 14.08.1997
(51) Int. Cl.: A61C 13/00, A61C 8/00

(54) **Verfahren zur Erhöhung der Lebensdauer von zahntechnischen Teilen**

(30) Priorität: 06.09.1996 DE 19636215
(71) Anmelder: Degussa Aktiengesellschaft, 60311 Frankfurt (DE)
(72) Erfinder: Lange, Gerlinde, 63505 Langenselbold (DE); Grau, Franz-Josef, Dr., 91077 Neunkirchen am Brand (DE); Kiese, Jürgen, Dr., 21698 Harsfeld (DE)

(57) **Zusammenfassung**

Zur Erhöhung der Lebensdauer von metallischen zahntechnischen Teilen gegenüber Ermüdungsbrüchen werden in die Oberflächenschicht dieser Teile Druckspannungen eingebracht, vorzugsweise durch Kugelstrahlen.

## Beschreibung

Die Erfindung betrifft ein Verfahren zur Erhöhung der Lebensdauer von metallischen zahntechnischen Teilen, wie Wurzelstiften, Implantaten, Prothesen oder Brücken, durch eine Oberflächenbehandlung dieser Teile.

Metallische Teile ertragen generell schwingende Belastungen auch dann nicht beliebig oft ohne zu Bruch zu gehen, wenn die Spannungsamplitude relativ klein gegenüber der im Zugversuch ermittelten Zugfestigkeit der entsprechenden Werkstoffe ist. Oft gehen metallische Bauteile auch dann noch zu Bruch, wenn die Spannungsamplitude kleiner als die Dehngrenze des eingesetzten Werkstoffes ist. Das Werkstoffverhalten wird also durch die Spannungsamplitude und die Häufigkeit ihrer Wiederholung bestimmt. Daneben wirken sich auch die Umgebungsbedingungen und die Geometrie der Teile auf die Schwingfestigkeit aus.

Im zahntechnischen Bereich werden viele Bauteile absichtlich (z.B. Federn) oder-unabsichtlich (z.B. Wurzelstifte) schwingend beansprucht. Dabei ergibt sich immer wieder das Problem, daß Bauteile, obwohl aus einer optimierten Legierung gefertigt und ausreichend dimensioniert, wie z.B. Wurzelstifte oder Konstruktionselemente, brechen, ohne daß im nachhinein Materialfehler oder Verarbeitungsfehler feststellbar sind. Vielmehr zeigen Untersuchungen, daß die Bauteile aufgrund von Materialermüdung, welche durch die Umgebungsbedingungen (Speichel) noch gefördert wird, versagt haben. Eine Möglichkeit, diese Ermüdungsbrüche zu vermeiden wäre eine stärkere Dimensionierung der Bauteile, um die lokalen Spannungen im Bauteil zu reduzieren. Dies ist jedoch oft aus Gründen der Platzsituation im Munde oder aus ästhetischen Gründen nicht möglich. Solche Brüche sind meist nur sehr schwer und mit großem Aufwand zu reparieren.

Im Fahrzeug- und Flugzeugbau ist das Kugelstrahlen zur Verbesserung der physikalischen Eigenschaften von Metallgegenständen schon seit vielen Jahren bekannt und wird dort erfolgreich eingesetzt. So wurde allgemein gefunden, daß bei vielen Teilen, welche einer Kugelstrahlung unterzogen wurden, eine erhöhte Ermüdungsfestigkeit festgestellt werden konnte. Die erhöhte Ermüdungsfestigkeit ist dabei auf die in die Oberflächenschicht eingebrachten Druckspannungen zurückzuführen, welche die Ausbildung von Rissen im Bauteil erschweren. So werden im Fahrzeugbau Differentialgetriebe für Fahrzeugachsen, Spindeln, Wellen, Druckringe sowie Verbindungsstangen kugelgestrahlt, um die Dauerfestigkeit zu erhöhen. Auch im Turbinenbau werden Schaufeln, welche im Verdichterteil der Turbine eingesetzt werden, oft kugelgestrahlt, um die Ermüdungsfestigkeit und damit die Lebensdauer des Bauteils zu erhöhen.

Solche Techniken sind im Dentalbereich bisher nicht angewendet worden. Spezielle Verfahren zur Verbesserung der Ermüdungsfestigkeit sind bisher auch noch nicht in Betracht gezogen worden.

Im Dentalbereich wird zum Beispiel das Sandstrahlen für das Reinigen von Gußteilen eingesetzt. Durch das Sandstrahlen wird aber mehr Material abgetragen als Verformung in die Oberflächenschicht eingebracht. Die Oberfläche der Teile wird daher nur aufgerauht. Diese mikroskopischen Defekte führen, da sie nicht von Druckspannungen überlagert werden, eher zu einer Abnahme der Ermüdungsfestigkeit dieser Teile.

Es war daher Aufgabe der vorliegenden Erfindung, ein Verfahren zur Erhöhung der Lebendauer von metallischen zahntechnischen Teilen, wie Wurzelstiften, Implantaten, Prothesen oder Brücken, durch eine Oberflächenbehandlung zu entwickeln.

Diese Aufgabe wird erfindungsgemäß dadurch gelöst, daß in die Oberflächenschicht dieser Teile durch mechanische Einwirkungen Druckspannungen eingebracht werden.

Vorzugsweise werden diese Druckspannungen durch Kugelstrahlen in die Oberflächenschicht eingebracht.

Überraschenderweise hat sich gezeigt, daß sich die Lebensdauer von aus üblichen Dentallegierungen gefertigten Teilen durch Einbringen von Druckspannungen in die Oberflächenschicht der Teile mittels Kugelstrahlen beträchtlich erhöht, so daß sich die Anzahl der Lastwechsel bis zum Bruch um über zwei Zehnerpotenzen erhöhen kann. Die auf der Oberfläche der kugelgestrahlten Teile zurückbleibenden Eindruckstellen können entweder mit geeigneten Mitteln, ohne Aufhebung der Druckspannungen in der Oberflächenschicht entfernt werden oder sie können zur Erhöhung der Retention, z.B. bei Wurzelstiften, belassen werden. Durch Sandstrahlen der Teile lässt sich eine Erhöhung der Ermüdungsfestigkeit nicht erzielen.

Folgende Beispiele sollen die Erfindung näher erläutern:

Probestäbe der Abmessung 40 mm Länge und 2,5 mm Durchmesser aus verschiedenen Werkstoffen wurden kugelgestrahlt mit Glaskugeln der Körnung 420 bis 590 µm. Der Strahldruck betrug 0,7 MPa bei einem Abstand Düse - Probe von 35 mm und einer Strahlzeit von 45 Sekunden. Die Probe rotierte dabei einmal pro Sekunde um ihre Achse.

Zur Beurteilung der Lebensdauer bzw. Dauerfestigkeit dieser Proben wurden sogenannte Wöhlerkurven bestimmt. Dazu werden mehrere Probestäbe aus dem gleichen Werkstoff bei konstanter Mittelspannung mit verschiedenen großen Spannungsamplituden bis zum Bruch schwingend beansprucht.

Die folgende Tabelle zeigt die gemessenen Werte an den kugelgestrahlten Proben, im Vergleich zu unbehandelten Proben für drei Werkstoffe. Man erkennt dabei die beträchtliche Erhöhung der Dauerfestigkeiten bzw. Lebensdauer der kugelgestrahlten Proben gegenüber den unbehandelten.

**Tabelle**

| | | Lastwechsel bis Bruch | |
|---|---|---|---|
| Werkstoff | angelegte Prüfspannung [MPa] | unbehandelte Proben | kugelgestrahlte Proben |
| 1 60 Au, 24,9 Pt, 15 Pd, 0,1 Ir | 200 | 89 000 | >11 000 000 |
| | 300 | 100 000 | 2 600 000 |
| | 350 | 61 000 | 2 200 000 |
| 2 Titan | 300 | 20 000 | >10 000 000 |
| | 350 | 10 000 | 300 000 |
| | 400 | 8 000 | 290 000 |
| 3 60 Pt 40 Au | 150 | 530 000 | >10 000 000 |
| | 200 | 450 000 | 8 300 000 |
| | 250 | 120 000 | 4 100 000 |
| | 300 | 85 000 | 2 500 000 |
| | 400 | 27 000 | 210 000 |

## Patentansprüche

1. Verfahren zur Erhöhung der Lebensdauer von metallischen zahntechnischen Teilen, wie Wurzelstifte, Implantate, Prothesen oder Brücken, durch eine Oberflächenbehandlung dieser Teile,
**dadurch gekennzeichnet,**
daß in die Oberflächenschicht dieser Teile durch mechanische Einwirkungen Druckspannungen eingebracht werden.

2. Verfahren nach Anspruch 1,
**dadurch gekennzeichnet,**
daß daß die Druckspannungen durch Kugelstrahlen in die Oberflächenschicht eingebracht werden.
